# EUROPÄISCHE PATENTSCHRIFT

(11) **EP 1 460 952 B1**
(45) Veröffentlichungstag und Bekanntmachung des Hinweises auf die Patenterteilung: **16.08.2006**
(21) Anmeldenummer: 01275124.4
(22) Anmeldetag: 31.12.2001
(51) Int. Cl.: A61B 17/70

(54) **VORRICHTUNG ZUR KUGELGELENKARTIGEN VERBINDUNG ZWEIER TEILE**
DEVICE FOR A BALL-AND-SOCKET-TYPE CONNECTION OF TWO PARTS
DISPOSITIF PERMETTANT DE RELIER DEUX PIECES A LA MANIERE D'UNE ARTICULATION SPHERIQUE

(43) Veröffentlichungstag der Anmeldung: 29.09.2004
(73) Patentinhaber: Synthes AG Chur, 7002 Chur (CH)
(72) Erfinder: SCHLÄPFER, Fridolin, CH-8750 Glarus (CH); DE FRANCESCHI, Renzo, CH-4433 Ramlinsburg (CH)
(74) Vertreter: Lusuardi, Werther
(86) Internationale Anmeldenummer: PCT/CH2001/000744
(87) Internationale Veröffentlichungsnummer: WO 2003/059182

(56) Entgegenhaltungen:
- WO-A-01/22893
- WO-A-02/09603
- FR-A- 2 810 533
- US-A- 5 733 286
- US-A- 6 063 090
- US-A- 6 086 588

## Beschreibung

Die Erfindung bezieht sich auf eine Vorrichtung zur kugelgelenkartigen Verbindung zweier Teile gemäss dem Oberbegriff des Patentanspruchs 1.

Beispielsweise werden für die interne Fixation der Wirbelsäule oder von Wirbelsäulenteilen Fixationsvorrichtung mit Gelenkverbindungen zwischen den Knochenfixationsmitteln, beispielsweise Pedikelschrauben und einem sich in Richtung der Wirbelsäule erstreckenden Längsträger verwendet. Zur stabilen Verankerung des gesamten Implantates müssen die Knochen- oder Pedikelschrauben einerseits fest mit den Wirbelkörpern und andererseits fest mit dem oder den Längsträgern verbindbar sein. Die Verbindung zwischen dem Kopf der Pedikelschrauben und dem Längsträger erfolgt üblicherweise mittels Klemmechanismen, welche auch unter verschiedenen Winkeln der Pedikelschraube zum Längsträger stabil sind. Die Klemmverbindung muss lösbar sein, damit das gesamte Implantat ohne grosse Gewebeöffnungen im Bereich der Wirbelsäule wieder entfernbar ist.

Eine solche Verbindung zwischen einer Knochenschraube und einem Stabilisationsstab zur internen Fixation von Wirbelkörpern ist aus der US 5,466,237 BYRD bekannt. Diese bekannte Erfindung weist eine Knochenverankerungsschraube mit einem Schraubenkopf auf, welcher an seiner dem Schraubenschaft zugewandten Seite kugelschichtförmig ausgestaltet ist und auf seiner dem Schraubenschaft abgewandten Seite endständig konvex ausgestaltet ist. Der kugelschichtförmige Teil der Schraube ist in einer Bohrung des Verbindungsteiles gelagert, wobei diese Bohrung einen konkaven und sich gegen den Schraubenschaft verjüngenden Abschnitt umfasst, wodurch zusammen mit dem Schraubenkopf eine kugelgelenkartige Verbindung zwischen der Knochenverankerungsschraube und dem Verbindungsteil hergestellt wird. Blockiert wird diese Verbindung durch Anziehen einer Mutter, welche über ein Aussengewinde am Verbindungsteil schraubbar ist und auf einen in den dazu vorgesehenen Kanal am Verbindungsteil eingelegten Längsträger drückt. Der Längsträger drückt auf seiner der Mutter gegenüberliegenden Seite auf den konvexen Teil der Knochenverankerungsschraube, so dass beim Anziehen der Mutter der Längsträger und die Knochenverankerungsschraube im Verbindungsteil blockiert werden.

Eine Knochenschraube mit einem Schraubenkopf, welcher auf seiner dem Schraubenschaft zugewandten Seite kugelschichtförmig ausgestaltet ist und auf seiner dem Schraubenschaft abgewandten Seite endständig sphärisch ausgebildet ist, ist aus der WO 01/03593 FRIGG bekannt. Bei dieser bekannten Knochenschraube können die Zentren der dem Schraubenschaft zugewandten Kugelschicht und der endständigen Kugelkappe bezüglich der Schraubenlängsachse beabstandet sein oder zusammenfallen.

Eine weitere Vorrichtung zur Verbindung einer Knochenschraube mit einem Längsträger, welche eine beschränkt separate Blockierung von Längsträger und Knochenschraube gestattet, ist aus der DE 43 07 576 BIEDERMANN bekannt.

Nachteilig an diesen bekannten Vorrichtungen ist die grosse Gesamtbauhöhe des die Gelenkverbindung aufweisenden Verbindungsteiles zwischen Knochenfixationsmittel und Längsträger.

Eine Vorrichtung zur Verbindung einer Knochenschraube mit einem Längsträger, welche durch die Ausgestaltung von Gelenkverbindung und deren Feststellmechanismus eine niedrigere Bauhöhe gestattet, ist aus der WO 01/22893 LOMBARDO bekannt. Bei dieser bekannten Vorrichtung ist der Schraubenkopf nur an seiner an den Schraubenschaft angrenzenden Seite sphärisch ausgestaltet und ist endständig abgeflacht. Auf seiner endständigen Oberfläche enthält der Schraubenkopf eine koaxiale, konische Kavität, worin das konvex ausgestaltete vordere Ende eines Klemmittels polyaxial bewegbar aufnehmbar und mittels eines Spannmittels gegen die konische Wand der Kavität pressbar ist. Das Spannmittel ist am hinteren Ende des Verbindungsteiles koaxial in dieses einschraubbar und drückt bei blockierter Vorrichtung mit seinem vorderen Ende gegen den in den dafür bestimmten Kanal im Verbindungsteil eingelegten Längsträger, welcher seinerseits auf das Klemmittel drückt. Nachteilig an dieser bekannten Vorrichtung ist, dass durch das Einpressen des konvexen, vorderen Endes des Klemmittels in die konische Kavität am Schraubenkopf der Knochenschraube ein Verkeilungseffekt mit einer die Knochenschraube parallel zur Zentralachse des Verbindungsteiles ausrichtenden Hebelkraft entsteht.

Andere Vorrichtungen zur Verbindung einer knochenschraube mit einem Längsträger sind aus US-A-6 086 588 und EP 1 153 577 (Basis für den Oberbegriff des Anspruchs 1) bekannt.

Hier will die Erfindung Abhilfe schaffen. Der Erfindung liegt die Aufgabe zugrunde, eine Vorrichtung zu schaffen, welche eine kugelgelenkartigen Verbindung zwischen zwei Teilen umfasst, wobei der Klemmechanismus ein Klemmittel umfasst, dessen vorderes Ende an der Oberfläche einer Vertiefung am Gelenkkopf ansteht, wodurch eine geringe Bauhöhe der Gelenkverbindung ermöglicht wird, und welches im blockierten Zustand eine polyaxial winklige Position der Zentralachsen der beiden Teile relativ zueinander gestattet.

Die Erfindung löst die gestellte Aufgabe mit einer Vorrichtung zur kugelgelenkartigen Verbindung zweier Teile, welche die Merkmale des Anspruchs 1 aufweist.

Die durch die Erfindung erreichten Vorteile sind im wesentlichen darin zu sehen, dass dank der erfindungsgemässen Vorrichtung:
- durch das Zusammenfallen des Gelenkzentrums mit dem Zentrum der sphärisch gewölbten Kontaktfläche am vorderen Ende des Klemmittels und/oder der Vertiefung am Gelenkkopf eine Blockierung der beiden Teile relativ zueinander unter verschiedenen Winkeln der Zentralachsen der beiden Teile ohne Auftreten von Hebelkräften ermöglicht wird; und
- wegen der Fixierung des Gelenkkopfes in der Kavität durch ein Klemmittel, dessen vorderes Ende in einer Vertiefung am Gelenkkopf zur Anlage bringbar ist, der Gelenkkopf niedriger aufgestaltet werden kann, wodurch die Vorrichtung geringere Abmessungen erhält.

Das Klemmittel kann in verschiedenen Ausführungsformen an seinem vorderen Ende je nach gewünschter Kontaktform mit der Vertiefung verschieden ausgestaltet sein:
- durch Ausgestaltung des vorderen Endes des Klemmittels als zur Zentralachse konzentrische Spitze ist ein punktförmiger Kontakt zwischen dem vorderen Ende und der Vertiefung erreichbar;
- durch Ausgestaltung des vorderen Endes des Klemmittels mit einer orthogonal zur Zentralachse abgeflachten Stirnfläche ist ein linienförmiger Kontakt zwischen dem vorderen Ende und der Vertiefung erreichbar; und
- durch eine sphärische Ausgestaltung des vorderen Endes des Klemmittels ist eine Kontaktfläche zwischen dem vorderen Ende und der Vertiefung herstellbar.

In weiteren Ausführungsformen kann die Vertiefung sphärisch oder konisch ausgestaltet sein, wodurch bei einer Paarung eines Klemmittels mit einem sphärisch ausgebildeten vorderen Ende eine Kontaktfläche oder ein linienförmiger Kontakt zwischen dem vorderen Ende des Klemmittels und der Vertiefung erreichbar ist.

In wiederum weiteren Ausführungsformen ist die Vertiefung mit einer zur Zentralachse konzentrischen, kreisförmigen Kante ausgestattet, welche mit dem sphärisch ausgestalteten vorderen Ende des Klemmittels ebenfalls einen linienförmigen Kontakt bildet.

Der Gelenkkopf ist rigid ausgebildet, so dass bei der Blockierung der Vorrichtung keine Aufweitung des Gelenkkopfes möglich ist.

In der bevorzugten Ausführungsform der erfindungsgemässen Vorrichtung ist
a) das erste Teil als Knochenfixationsmittel, beispielsweise als Pedikelschraube oder als Pedikelhaken ausgestaltet, deren unteres Segment zur Fixation des Knochenfixationsmittels an einem Knochen dient; und
b) das zweite Teil als Verbindungsteil zwischen einem Längsträger und dem Knochenfixationsmittel ausgestaltet, welches einen quer zur Zentralachse angeordneten Kanal zur Aufnahme eines Längsträgers und eine koaxial zur Zentralachse angeordnete Zentralbohrung mit einem Innengewinde umfasst.

Ferner umfasst die Vorrichtung Spannmittel zur lösbaren Blockierung eines im Kanal eingelegten Längsträgers, so dass die Vorrichtung beispielsweise als Wirbelsäulenfixationsvorrichtung anwendbar ist.

Die Klemmittel können direkt fixier- respektive lösbar sein und beispielsweise als Klemmschraube ausgestaltet sein, welche beispielsweise in das Innengewinde in der Zentralbohrung im Verbindungsteil einschraubbar ist. Dadurch wird ermöglicht, dass die Blockierung oder das Lösen des Knochenfixationsmittels mittels der Klemmschraube im Verbindungsteil unabhängig von der Blockierung oder des Lösens des Längsträgers mittels der Spannmittel erfolgen kann.

In der erfindungsgemässen Vorrichtung ist der Kanal seitlich offen ausgestaltet. Eine Verschlusskappe, welche parallel zur Zentralachse über das Verbindungsteil schiebbar ist und eine zur Zentralachse parallele Lasche aufweist, ermöglicht beim Anziehen der Spannmittel das Verschliessen der Kanalöffnung und das Einklemmen des Längsträgers im Kanal zwischen der Kanalwand und der Lasche. Der Kanal ist bezüglich der Zentralachse vorzugsweise gegen die Kanalöffnung versetzt. Dadurch wird ermöglicht, dass die Klemmschraube neben dem Kanal in das Verbindungsteil eingeschraubt werden kann, so dass eine erheblich geringere Bauhöhe des Verbindungsteiles möglich ist.

In einer weiteren Ausführungsform der erfindungsgemässen Vorrichtung umfasst der Gelenkkopf zwei parallele und zur Zentralachse parallele Abflachungen, welche einen Abstand B zueinander aufweisen während in der Verengung eine zur Zentralachse parallele Nute mit einer Weite W angebracht ist, welche die Öffnung der Kavität am unteren Ende des zweiten Teiles senkrecht zur Weite W derart vergrössert, dass der Gelenkkopf mit zu den Seitenwänden der Nute parallelen Abflachungen vom unteren Ende des zweiten Teiles her in die Kavität ein- oder ausführbar ist, während bei gegenüber den Seitenwänden der Nute gedrehten Abflachungen der Gelenkkopf durch die Auflage in der Kavität axial gegen das untere Ende des zweiten Teiles gesichert ist.

Weitere vorteilhafte Ausgestaltungen der Erfindung sind in den abhängigen Ansprüchen gekennzeichnet.

Die Erfindung und Weiterbildungen der Erfindung werden im folgenden anhand der teilweise schematischen Darstellungen mehrerer Ausführungsbeispiele noch näher erläutert.

Es zeigen:
Fig. 1 einen Längsschnitt durch eine Ausführungsform der erfindungsgemässen Vorrichtung;
Fig. 2a einen Ausschnitt des Klemmittels gemäss einer Ausführungsform der erfindungsgemässen Vorrichtung;
Fig. 2b einen Ausschnitt des Klemmittels gemäss einer anderen Ausführungsform der erfindungsgemässen Vorrichtung;
Fig. 3 einen Längsschnitt durch eine weitere Ausführungsform der erfindungsgemässen Vorrichtung;
Fig. 4a einen Schnitt durch den Gelenkkopf einer Ausführungsform der erfindungsgemässen Vorrichtung;
Fig. 4b einen Schnitt durch den Gelenkkopf einer anderen Ausführungsform der efindungsgemässen Vorrichtung;
Fig. 5 einen Schnitt durch eine weitere Ausführungsform der erfindungsgemässen Vorrichtung;
Fig. 6 eine Ansicht von oben auf die in Fig. 5 dargestellte Ausführungsform der erfindungsgemässen Vorrichtung;
Fig. 7 eine Ansicht von unten auf die in den Fig. 5 und 6 dargestellte Ausführungsform der erfindungsgemässen Vorrichtung;
Fig. 8 einen Schnitt durch eine nicht erfindungsgemässe Vorrichtung;
Fig. 9 einen Schnitt durch eine weitere nicht erfindungsgemässe Vorrichtung;
Fig. 10 eine Explosionsdarstellung der in den Fig. 5, 6 und 7 dargestellten Ausführungsform der erfindungsgemässen Vorrichtung; und
Fig. 11 eine Explosionsdarstellung eines Teils einer nicht erfindungsgemässen Vorrichtung zusammen mit einer Befestigungsvorrichtung.

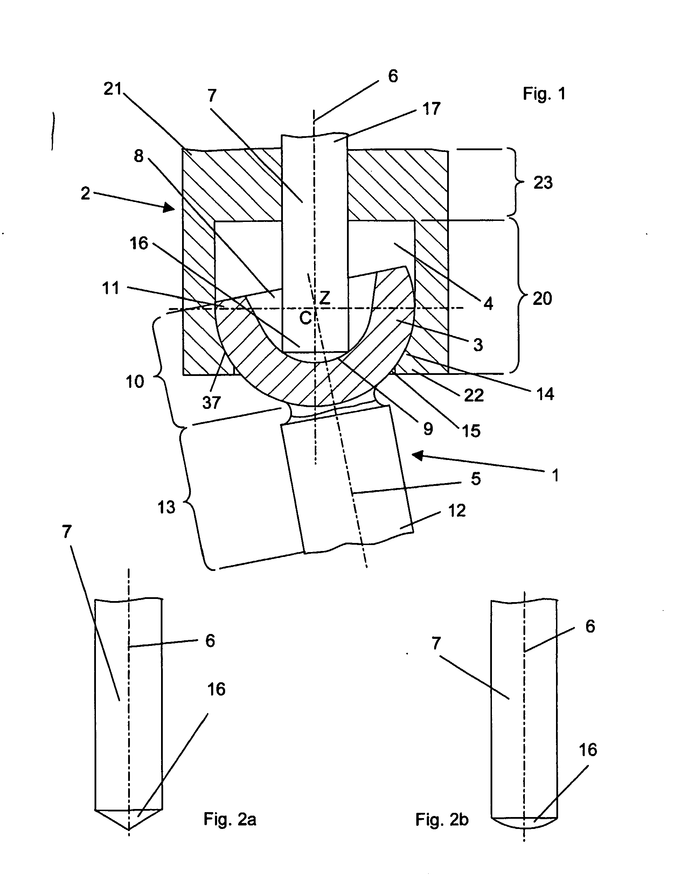

In Fig. 1 dargestellt ist das erste Teil 1, das zweite Teil 2 mit der Kavität 4 sowie das Klemmittel 7. Das erste Teil 1 weist eine Zentralachse 5, ein oberes Ende 11, ein unteres Ende 12, sowie beim oberen Ende 11 ein erstes Gelenksegment 10 auf, welches als kugelsegmentförmiger Gelenkkopf 3 ausgebildet ist, wobei das Kugelzentrum auf der Zentralachse 5 liegt und das Gelenkzentrum Z bildet. Der Gelenkkopf 3 ist beim oberen Ende 11 des ersten Teils 1 abgeflacht. Ferner umfasst der Gelenkkopf 3 eine Vertiefung 8, welche teilweise sphärisch ausgestaltet ist, eine kugelkappenförmige Oberfläche 9 aufweist und konisch in das obere Ende 11 des ersten Teiles 1 mündet. Die kugelkappenförmige, das Zentrum C aufweisende Oberfläche 9 der Vertiefung 8 ist so ausgebildet, dass das Zentrum C auf der Zentralachse 5 liegt und mit dem Gelenkzentrum Z zusammenfällt. Das zweite Teil 2 weist eine Zentralachse 6, ein oberes Ende 21, ein unteres Ende 22 sowie axial aneinandergrenzend ein oberes Segment 23 und eine beim unteren Ende 22 angeordnete, das zweite Gelenksegment 20 bildende Kavität 4 auf. Die Kavität 4 ist koaxial zur Zentralachse 6 angeordnet und wird am unteren Ende 22 des zweiten Teiles 2 durch eine Verengung 14 verjüngt. Die Verengung 14 weist gegen das Innere der Kavität 4 gerichtet eine kugelzonenförmige, zur kugelzonenförmigen Oberfläche des Gelenkkopfes 3 komplementäre Auflage 37 mit dem Gelenkzentrum Z auf, so dass die beiden Gelenksegmente 10;20 relativ zueinander um drei senkrecht aufeinander stehende Achsen drehbar sind. Am unteren Ende 22 des zweiten Teiles 2 weist die Kavität 4 eine zur Zentralachse 6 koaxiale Öffnung 15 auf, wodurch das untere Segment 13 des ersten Teiles 1 durchführbar ist. Das Klemmittel 7 ist koaxial zur Zentralachse 6 angeordnet, weist ein vorderes und ein hinteres Ende 16;17 auf und ist koaxial zur Zentralachse 6 verschiebbar angeordnet. Das vordere Ende 16 des Klemmittels 7 ist kreiszylindrisch ausgebildet und auf der kugelkappenförmigen Oberfläche 9 der Vertiefung 8 zur Anlage bringbar. Durch Anpressen des vorderen Endes 16 auf die Oberfläche 9 der Vertiefung 8 ist der Gelenkkopf 3 in der Kavität 4 blockierbar, wobei wegen der Identität des Zentrums C der kugelkappenförmigen Oberfläche 9 der Vertiefung 8 mit dem Gelenkzentrum Z die beiden Teile 1;2 mit polyaxial winkliger Anordnung ihrer Zentralachsen 5;6 blockierbar sind.

Die Fig. 2a und 2b zeigen jeweils verschiedene Ausführungsformen des Klemmittels 7, wobei in Fig. 2a das vordere Ende 16 des Klemmittels 7 eine zur Zentralachse 6 koaxiale Spitze aufweist, während in Fig. 2b das vordere Ende 16 des Klemmittels 7 sphärisch konvex ausgestaltet ist.

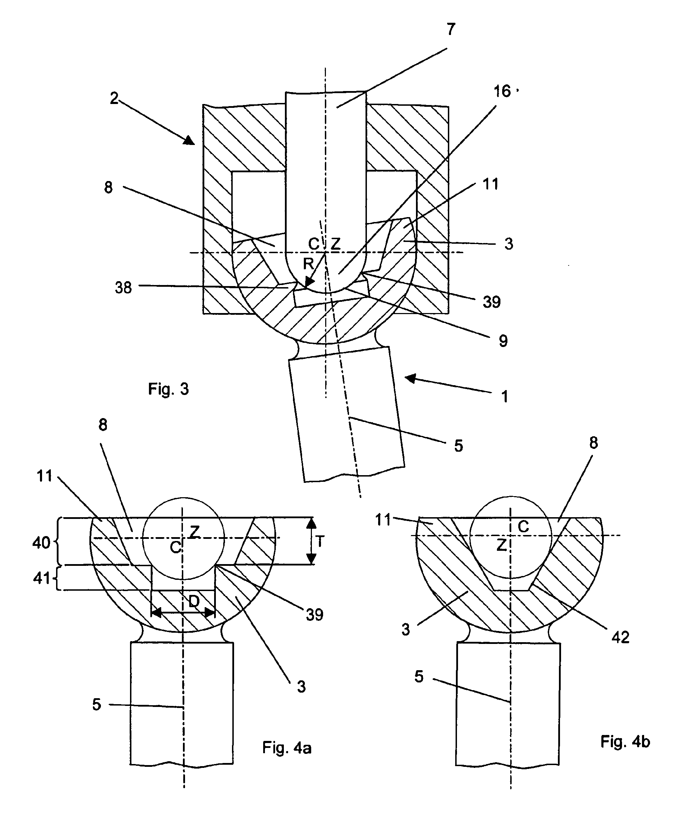

Die in Fig. 3 dargestellte Ausführungsform der erfindungsgemässen Vorrichtung unterscheidet sich von der in Fig. 1 dargestellten Ausführungsform nur darin, dass die Vertiefung 8 im Gelenkkopf 3 und das vordere Ende 16 des Klemmittels 7 anders ausgestaltet sind. Das vordere Ende 16 des Klemmittels 7 ist halbkugelförmig mit dem auf der Zentralachse 6 liegenden Zentrum C ausgestaltet und weist einen Radius R auf. Die Vertiefung 8 ist konisch mit einer zur Zentralachse 6 konzentrischen, kreisringförmigen Erhebung 38 ausgestaltet. Die Erhebung 38 weist gegen das Innere der Vertiefung 38 gerichtet eine kreisförmige, scharfe Kante 39 auf. Der axiale Abstand der Kante 39 zur oberen Ende 11 des ersten Teiles 1 und der Durchmesser der kreisförmigen Kante 39 sind derart ausgestaltet, dass die Kante 39 auf einer imaginären Kugeloberfläche mit dem auf der Zentralachse 6 liegenden Zentrum C und dem Radius R liegt.

Die in Fig. 4a dargestellte Ausführungsform des Gelenkkopfes 3 unterscheidet sich von den oben beschriebenen Ausführungsformen nur dadurch, dass die Vertiefung 8 am oberen Ende 11 des ersten Teiles 1 einen konischen, koaxialen Abschnitt 40 und in einer Tiefe T vom oberen Ende 11 her gemessen, axial daran angrenzend einen kreiszylindrischen Abschnitt 41 mit dem Durchmesser D aufweist. Der Durchmesser D ist so gewählt, dass die Vertiefung 8 durch den kreiszylindrischen Abschnitt 41 in der Tiefe T verengt wird, so dass durch den Übergang zwischen dem konischen Abschnitt 4 und dem kreiszylindrischen Abschnitt 41 eine zur Zentralachse 6 konzentrische, kreisförmige Kante 39 gebildet wird. Ferner sind der Durchmesser D und die Tiefe T so bemessen, dass die kreisförmige Kante 39 auf einer imaginären Kugeloberlfäche mit dem auf der Zentralachse 6 liegenden Zentrum C und dem Radius R (Fig. 3) zusammenfällt.

In Fig. 4b ist eine Ausführungsform des Gelenkkopfes 3 mit einer konischen Vertiefung 8 dargestellt. Der Durchmesser des Konus 42 am oberen Ende 11 des ersten Teiles 1 und der Konuswinkel des Konus 42 sind so bemessen, dass der Konus 42 eine imaginäre Kugeloberfläche mit dem auf der Zentralachse 6 liegenden Zentrum C und dem Radius R (Fig. 3) berührt.

Durch eine Ausgestaltung der Vertiefung 8 und des vorderen Endes 16 des Klemmittels 7 gemäss einer der Fig. 3, 4a oder 4b ist ein linienförmiger Kontakt zwischen dem Klemmittel 7 und der Vertiefung 8 erreichbar.

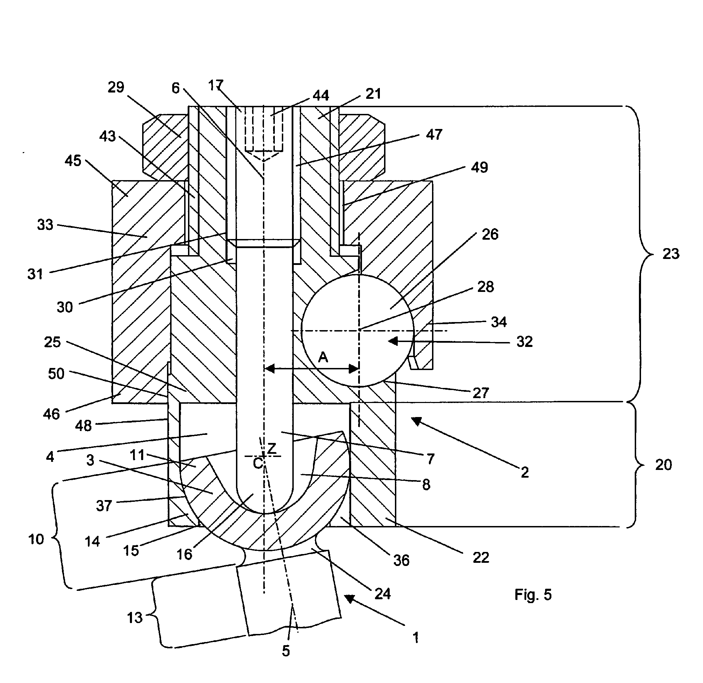

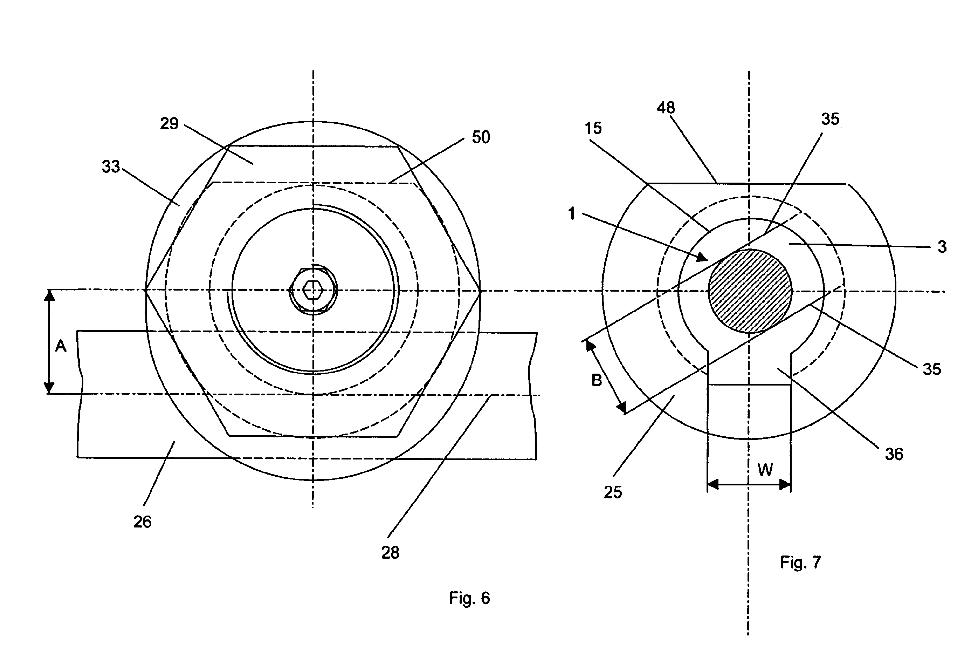

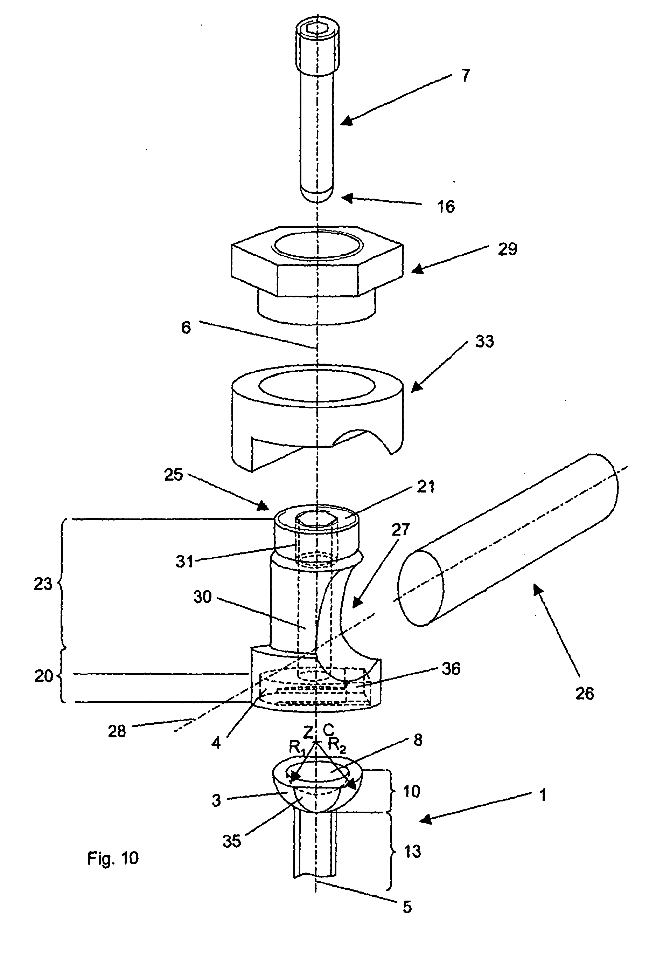

In den Fig. 5 bis 7 und 10 ist eine Ausführungsform der erfindungsgemässen Vorrichtung dargestellt, welche als erstes Teil 1 ein als Pedikelschraube ausgeführtes Knochenfixationsmittel 24 und als zweites Teil 2 ein Verbindungsteil 25 zur Verbindung eines Längsträgers 26 mit dem Knochenfixationsmittel 24 umfasst. Das als Pedikelschraube ausgeführte Knochenfixationsmittel 24 umfasst einen Gewindeschaft als unteres Segment 13 und einen Schraubenkopf als Gelenkkopf 3. Der Schraubenkopf ist auf seiner dem Schraubenschaft zugewandten Seite teilweise sphärisch mit dem Radius R₂ ausgestaltet, wobei das auf der Zentralachse 6 liegende Zentrum dieses Kugelsegmentes das Gelenkzentrum Z bildet. Ferner enthält der Gelenkkopf 3 eine Vertiefung 8, welche eine teilweise sphärische Oberfläche 9 mit dem Zentrum C und dem Radius R₁ aufweist und konisch in die Aussenfläche am oberen Ende 1 des ersten Teiles 1 mündet. Das Zentrum C fällt mit dem Gelenkzentrum Z zusammen, so dass der Schraubenkopf so im Verbindungsteil 25 blockierbar ist, dass die Zentralachse 5 des ersten Teiles 1 und die Zentralachse 5 des zweiten Teiles 2 polyaxial winklig zueinander verlaufen können.

Die als zweites Gelenksegment 20 dienende Kavität 4 im Verbindungsteil 25 weist eine ebenfalls sphärische, das Gelenkzentrum Z aufweisende Auflage 37 an der Verengung 14 auf, so dass der sphärisch ausgestaltete Schraubenkopf auf der Auflage 37 um das Gelenkzentrum Z polyaxial drehbar gelagert ist. Die Klemmittel 7 sind als Klemmschraube ausgestaltet, welche am hinteren Ende 17 ein Aussengewinde 47 sowie Mittel 44 zur Aufnahme eines Schraubendrehers aufweist, und in ein zu diesem Aussengewinde 47 komplementäres Innengewinde 31 in der Zentralbohrung 30 im Verbindungsteil 25 einschraubbar ist. Das vordere Ende 16 des Klemmittels 7 ist sphärisch ausgestaltet und auf der sphärischen Oberfläche 9 in der Vertiefung 8 am Schraubenkopf zur Anlage bringbar.

Der Gelenkkopf 3 ist mit zwei zueinander parallelen und zur Zentralachse 5 parallelen Abflachen 35 ausgestattet, welche einen Abstand B zueinander aufweisen. Ferner weist die Kavität 4 eine zur Zentralachse 6 parallele Nute 36 mit einer Breite W ≥ B auf. Die Tiefe der Nute 36 ist derart ausgestaltet, dass die Öffnung 15 der Kavität 4 am unteren Ende 22 des zweiten Teiles 2 senkrecht zur Breite B so vergrössert wird, dass der Gelenkkopf 3 mit zu den Seitenwänden der Nute 36 parallelen Abflachungen 35 vom unteren Ende 22 des zweiten Teiles 2 her in die Kavität 4 ein- oder daraus ausführbar ist. Durch Drehen des ersten Teiles 1 um die Zentralachse 5 werden die Abflachungen 35 am Gelenkkopf 3 gegenüber den Seitenwänden der Nute 36 verdreht, so dass der Gelenkkopf 3 durch die Verengung 14 in der Kavität 4 axial gegen das untere Ende 22 des zweiten Teiles 2 arretierbar ist.

Der Kanal 27 zur Aufnahme des Längsträgers 26 ist im Verbindungsteil 25 mit quer zur Zentralachse 6 verlaufender Kanalachse 28 angebracht. Ferner ist der Kanal 27 seitlich am Verbindungsteil 25 offen und weist dort eine Kanalöffnung 32 auf. Der Kanal 27 ist dabei so am Verbindungsteil 25 angebracht, dass die Kanalachse 28 von der Zentralachse 6 weg zur Kanalöffnung 32 hin um einen Abstand A verschoben ist. Zum Fixieren des Längsträgers 26 im Kanal 27 wird eine Verschlusskappe 33 koaxial zur Zentralachse 6 über das Verbindungsteil 25 geschoben. Die Verschlusskappe 33 umfasst eine zur Zentralachse 6 parallele Lasche 34 mittels welcher bei über das Verbindungsteil 25 geschobener Verschlusskappe 33 die Kanalöffnung 32 verschliessbar ist und ein im Kanal 27 eingelegter Längsträger 26 im Kanal 27 arretierbar ist. Die Verschlusskappe 33 ist mittels des Spannmittels 29, welches hier als Mutter ausgestaltet ist und koaxial zur Zentralachse 6 auf ein am oberen Ende 21 des Verbindungsteiles 25 angebrachtes Aussengewinde 43 schraubbar ist, koaxial zur Zentralachse 6 gegen das untere Ende 22 des Verbindungsteiles 25 pressbar, so dass ein im Kanal 27 eingelegter Längsträger 26 im Kanal 27 lösbar blockierbar ist.

Zwischen dem Verbindungsteil 25 und der Verschlusskappe 33 ist eine Verdrehsicherung angebracht. Diese besteht darin, dass das Verbindungsteil 25 auf einem Stück seiner zur Zentralachse 6 parallelen Länge eine Abflachung 48 aufweist und die zur Zentralachse 6 koaxiale Zentralöffnung 49 der Verschlusskappe 33 eine zur Abflachung 48 komplementäre Querschnittsverengung 50 aufweist. Bei über das Verbindungsteil 25 geschobener Verschlusskappe 33 kommt diese Querschnittsverengung 50 an der Abflachung 48 zur Anlage, so dass eine Verdrehung der Verschlusskappe 33 relativ zum Verbindungsteil 25 um die Zentralachse 6 verhindert wird.

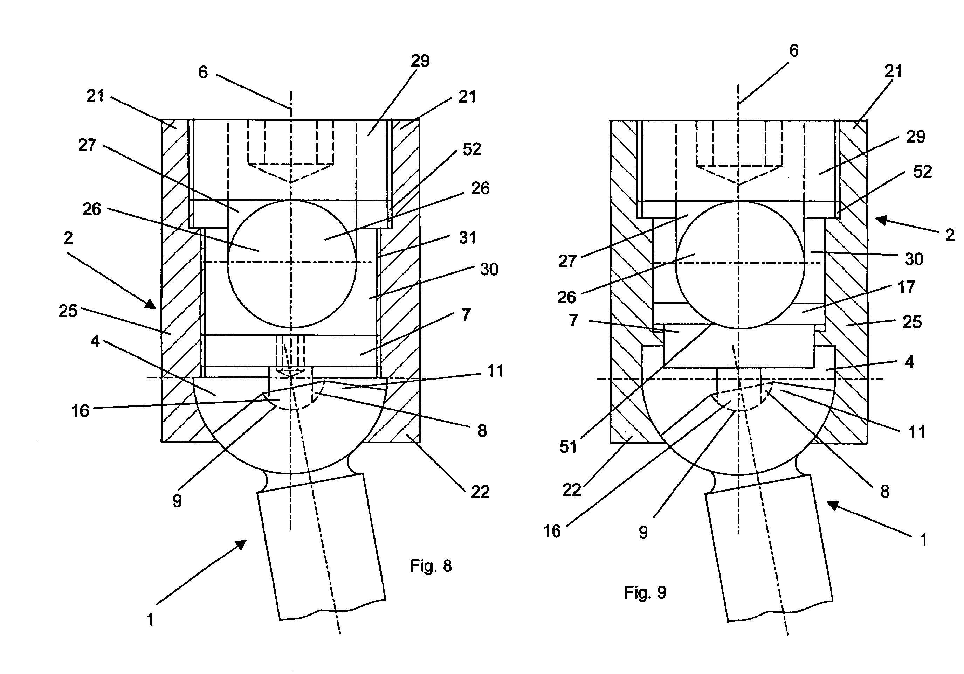

Die in Fig. 8 dargestellte nicht erfindungsgemässe Vorrichtung unterscheidet sich von der in den Fig. 1 und 3 dargestellten Vorrichtung nur darin, dass das hier ebenfalls als Verbindungsteil 25 ausgestaltete zweite Teil 2 einen gegen das obere Ende 21 des Verbindungsteiles 25 offenen Kanal 27 zur Aufnahme eines Längsträgers 26 umfasst. Das Klemmittel 7 ist koaxial zur Zentralachse 6 in das Innengewinde 31 in der Zentralbohrung 30 im Verbindungsteil 25 einschraubbar, so dass sein vorderen Ende 16 auf die Oberfläche 9 der Vertiefung 8 am oberen Ende 11 des ersten Teiles 1 pressbar ist. Das Klemmittel 7 ist bei montierter Vorrichtung zwischen dem in den Kanal 27 eingelegten Längsträger 26 und der Kavität 4 im zweiten Teil 2 angeordnet. Im Kanal 27 blockiert wird der Längsträger 26 durch ein als Stiftschraube ausgebildetes Spannmittel 29, welches nach dem Einlegen des Längsträgers 26 in den Kanal 27 vom oberen Ende 21 des Verbindungsteiles 25 her in das Innengewinde 52 einschraubbar ist. Das Innengewinde 52 und das Innengewinde 31, welche beide koaxial zur Zentralachse 6 in der Zentralbohrung 30 angeordnet sind können auch als einziges Innengewinde ausgestaltet sein.

Die in Fig. 9 dargestellte nicht erfindungsgemässe Vorrichtung unterscheidet sich von der in Fig. 8 dargestellten Vorrichtung nur dadurch, dass das Innengewinde 52 in der Zentralbohrung 30 vom oberen Ende 21 des Verbindungsteiles 25 nur soweit reicht, dass mittels des Spannmittels 29 ein im Kanal 27 eingelegter Längsträger 26 im Kanal 27 blockierbar ist. Zwischen dem mit dem Innengewinde 52 versehenen Längsabschnitt der Zentralbohrung 30 und der Kavität 4 ist die Zentralbohrung 30 kreiszylindrisch ausgestaltet, so dass das Klemmittel 7 parallel zur Zentralachse 6 in der Zentralbohrung 30 verschiebbar ist. Das Klemmittel 7 ist an seinem hinteren Ende 17 mit einer quer zur Zentralachse 6 verlaufenden Kerbe 51 ausgestattet. Bei montierter Vorrichtung liegt der in den Kanal 27 eingelegte Längsträger 26 in der Kerbe 51 auf, so dass beim Anziehen der Spannmittels 29 dieses auf den Längsträger 26 drückt, welcher seinerseits auf das Klemmittel 7 drückt. Das Klemmittel 7 wird parallel zur Zentralachse 6 gegen das vordere Ende 22 des Verbindungsteiles 25 verschoben und wird mit seinem vorderen Ende 16 auf die Oberfläche 9 der Vertiefung 8 am oberen Ende 11 des ersten Teiles 1 gepresst. Damit ist erreichbar, dass durch Anziehen des Spannmittels 29 der Längsträger 26 und das erste Teil 1 simultan in der Vorrichtung blockierbar sind.

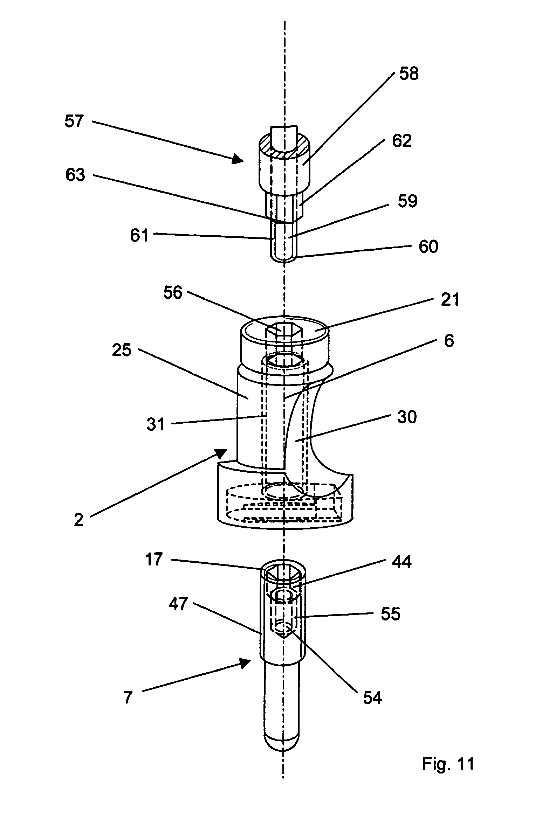

Fig. 11 zeigt das als Verbindungsteil 25 ausgestaltete zweite Teil 2 zusammen mit einem Klemmittel 7 und einer Befestigungsvorrichtung 57 für das zweite Teil 2. Die Befestigungsvorrichtung 57 umfasst einen hülsenförmigen Halter 58 und einen im Halter 58 koaxial zur Zentralachse 6 verschiebbaren Gewindebolzen 59. Der Halter 58 weist an seinem vorderen Ende 63 einen zur Zentralachse 6 parallelen Aussensechskant 62 auf, welcher in den dazu komplementären Innensechskant 56 am hinteren Ende 21 des Verbindungsteiles 25 gesteckt wird. Der Gewindebolzen 59 weist an seinem vorderen Ende 60 koaxial zur Zentralachse 6 ein Aussengewinde 61 auf, so dass der Gewindebolzen 59 in ein dazu komplementäres Innengewinde 55 in einer vom hinteren Ende 17 des Klemmittels 7 koaxial zur Zentralachse 6 in dieses eindringenden Bohrung 54 einschraubbar ist und mit der Befestigungsvorrichtung verbindbar ist. Das Klemmittel 7, welches mittels einem axial bis zu seinem hinteren Ende 17 reichenden Aussengewinde 47 versehen ist und in ein dazu komplementäres Innengewinde 31 in der Zentralbohrung 30 im Verbindungsteil 25 schraubbar ist, wird in der Zentralbohrung 30 im Verbindungsteil 25 vollständig zurückgedreht. Ferner weist das Klemmittel 7 an seinem hinteren Ende 17 koaxial zur Zentralachse 6 Mittel 44 zur Aufnahme eines Schraubendrehers auf, welche hier als Innensechskant ausgestaltet sind. Dieser Innensechskant weist in der hier dargestellten nicht erfindungsgemässen Vorrichtung eine Schlüsselweite auf, welche dem Aussendurchmesser des Innengewindes 55 entspricht. Der am hinteren Ende 21 des Verbindungsteiles 25 koaxial zur Zentralachse 6 in der Zentralbohrung 30 angebrachte Innensechskant 56 weist eine Schlüsselweite auf, welche dem Innendurchmesser des Innengewindes 31 in der Zentralbohrung 30 entspricht. In anderen Vorrichtungen können die Mittel 44 zur Aufnahme eines Schraubendrehers am Klemmittel 7 auch eine andere, zur Übertragung eines Drehmomentes geeignete Form aufweisen. Dasselbe gilt für den Aussensechskant 62 am vorderen Ende 63 des Halters 58 und den dazu komplementären Innensechskant 56 am hinteren Ende 21 des Verbindungsteiles 25.

## Patentansprüche

1. Vorrichtung zur kugelgelenkartigen Verbindung zweier Teile umfassend
A) ein erstes Teil (1) und ein zweites Teil (2), welche kugelgelenkartig miteinander verbunden sind, wobei
B) das erste Teil (1) eine erste Zentralachse (5), ein erstes Gelenksegment (10) mit einem oberen Ende (11) und ein bezüglich der Gelenkverbindung unspezifisches, unteres Segment (13) umfasst;
C) das zweite Teil (2) eine zweite Zentralachse (6), ein zweites Gelenksegment (20) mit einem unteren Ende (22) und ein bezüglich der Gelenkverbindung unspezifisches, oberes Segment (23) mit einem oberen Ende (21) umfasst;
D) das erste Gelenksegment (10) als Gelenkkopf (3) und das zweite Gelenksegment (20) als eine den Gelenkkopf (3) aufnehmende, zur zweiten Zentralachse (6) konzentrische Kavität (4) mit einer gegen das untere Ende (22) des zweiten Teiles (2) offenen Öffnung (15) zur Durchführung des unteren Segmentes (13) des ersten Teiles (1) ausgestaltet sind;
E) die zur zweiten Zentralachse (6) konzentrische Kavität (4) gegen das untere Ende (22) des zweiten Teiles (2) hin durch eine Verjüngung (14) verjüngt wird; und
F) die Verjüngung (14) eine gegen das Innere der Kavität (4) gerichtete Auflage (37) für den Gelenkkopf (3) aufweist;
G) mindestens eines der Gelenksegmente (10;20) im Kontaktbereich der Gelenksegmente (10;20) sphärisch mit dem Gelenkzentrum Z ausgestaltet ist;
H) die gelenkartige Verbindung zwischen den beiden Gelenksegmenten (10;20) mittels eines mit dem zweiten Gelenksegment (20) verbindbaren, ein vorderes und ein hinteres Ende (16;17) aufweisenden, koaxial zur zweiten Zentralachse (6) angeordneten Klemmittels (7) lösbar fixierbar ist;
I) der Gelenkkopf (3) am oberen Ende (11) eine Vertiefung (8) aufweist, worin das vordere Ende (16) des Klemmittels (7) zur Anlage bringbar ist; und
K) die Vertiefung (8) und/oder das vordere Ende (16) des Klemmittels (7) eine mindestens partiell sphärische, das Zentrum C aufweisende Oberfläche (9) aufweist, wobei
L) im blockierten Zustand der Vorrichtung das Gelenkzentrum Z und das Zentrum C identisch sind;
M) das zweite Teil (2) ein Verbindungsteil (25) zur Verbindung eines Längsträgers (26) mit dem ersten Teil (1) ist; und
N) das Verbindungsteil (25) einen quer zur zweiten Zentralachse (6) angeordneten Kanal (27) mit einer Kanalachse (28) zur Aufnahme eines Längsträgers (26) umfasst, **dadurch gekennzeichnet, dass**
O) dass die Kanalachse (28) zur zweiten Zentralachse (6) seitlich so weit verschoben ist, dass das Klemmittel (7) seitlich nicht in den Kanal (27) ragt; und
P) die Vorrichtung eine Verschlusskappe (33) umfasst, welche koaxial zur zweiten Zentralachse (6) über das Verbindungsteil (25) schiebbar ist, mittels am Verbindungsteil (25) angeordneten Spannmitteln (29) gegen das untere Ende (22) des Verbindungsteiles (25) pressbar ist und eine zur zweiten Zentralachse (6) parallele Lasche (34) umfasst, wobei
Q) die Lasche (34) bei montierter Verschlusskappe (33) einen im Kanal (27) eingelegten Längsträger (26) seitlich umschliesst.

2. Vorrichtung nach Anspruch 1, **dadurch gekennzeichnet, dass** das zweite Teil (2) eine koaxial zur zweiten Zentralachse (6) angeordnete Zentralbohrung (30) umfasst.

3. Vorrichtung nach Anspruch 2, **dadurch gekennzeichnet, dass** am Verbindungsteil (25) Spannmittel (29) zur lösbaren Blockierung eines im Kanal (27) einführbaren Längsträgers (26) angeordnet sind.

4. Vorrichtung nach Anspruch 2 oder 3, **dadurch gekennzeichnet, dass** die Zentralbohrung (30) ein Innengewinde (31) aufweist und dass das Klemmittel (7) eine Klemmschraube ist, welche in das Innengewinde (31) im Verbindungsteil (25) einschraubbar ist.

5. Vorrichtung nach einem der Ansprüche 1 bis 4, **dadurch gekennzeichnet, dass** der Kanal (27) seitlich offen ist und seitlich eine quer zur zweiten Zentralachse (6) und quer zur Kanalachse (28) angeordnete Kanalöffnung (32) aufweist.

6. Vorrichtung nach Anspruch 2, **dadurch gekennzeichnet, dass** das Klemmittel (7) in der Zentralbohrung (30) axial verschiebbar ist.

7. Vorrichtung nach Anspruch 2, **dadurch gekennzeichnet, dass** die Zentralbohrung (30) ein Innengewinde (31) und das Klemmittel (7) ein Aussengewinde (47) umfasst, wobei das Innengewinde (31) so lang ist, dass das Klemmittel (7) in Anlage mit dem ersten Teil (1) bringbar ist.

8. Vorrichtung nach den Ansprüchen 5 bis 7, **dadurch gekennzeichnet, dass** die Zentralbohrung (30) am oberen Ende (21) des Verbindungsteiles (25) ein Innengewinde (31) umfasst und dass das Spannmittel (29) auf einem in den Kanal (27) eingelegten Längsträger (26) zur Anlage bringbar ist.

9. Vorrichtung nach einem der Ansprüche 1 bis 8, **dadurch gekennzeichnet, dass** das Verbindungsteil (25) an seinem oberen Ende (21) ein Aussengewinde (43) umfasst und dass das Spannmittel (29) eine Mutter ist, welche über das Aussengewinde (43) schraubbar ist, so dass das Spannmittel (29) auf einem in den Kanal (27) eingelegten Längsträger (26) zur Anlage bringbar sind.

10. Vorrichtung nach einem der Ansprüche 1 bis 9, **dadurch gekennzeichnet, dass** Klemmittel (7) an seinem vorderen Ende (16) eine zur zweiten Zentralachse (6) konzentrische Spitze aufweist.

11. Vorrichtung nach einem der Ansprüche 1 bis 10, **dadurch gekennzeichnet, dass** das Klemmittel (7) an seinem vorderen Ende (16) eine zur zweiten Zentralachse (6) orthogonale, flache Stirnfläche aufweist.

12. Vorrichtung nach einem der Ansprüche 1 bis 11, **dadurch gekennzeichnet, dass** das Klemmittel (7) an seinem vorderen Ende (16) sphärisch ausgestaltet ist.

13. Vorrichtung nach Anspruch 12, **dadurch gekennzeichnet, dass** das Klemmittel (7) an seinem vorderen Ende (16) sphärisch mit dem Zentrum C und dem Radius R ausgestaltet ist.

14. Vorrichtung nach einem der Ansprüche 1 bis 13, **dadurch gekennzeichnet, dass** die Vertiefung (8) sphärisch mit dem Zentrum C ausgestaltet ist.

15. Vorrichtung nach Anspruch 14, **dadurch gekennzeichnet, dass** die Vertiefung (8) als Konus (42) ausgestaltet ist, wobei der Durchmesser des Konus (42) am oberen Ende des ersten Teiles (1) und der Konuswinkel so bemessen sind, dass der Konus (42) eine imaginäre Kugeloberfläche mit dem Zentrum C und dem Radius R berührt.

16. Vorrichtung nach Anspruch 15, **dadurch gekennzeichnet, dass** die Vertiefung (8) mit einer zur zweiten Zentralachse (6) konzentrischen, kreisförmigen Kante (39) ausgestaltet ist, wobei die Kante (39) auf einer imaginären Kugeloberfläche mit dem Zentrum C und dem Radius R liegt.

17. Vorrichtung nach einem der Ansprüche 1 bis 16, **dadurch gekennzeichnet, dass** der Gelenkkopf (3) rigid ist, so dass bei der Blockierung der Vorrichtung keine Aufweitung des Gelenkkopfes (3) möglich ist.

18. Vorrichtung nach einem der Ansprüche 1 bis 17, **dadurch gekennzeichnet, dass** mindestens eine der sphärisch ausgebildeten Flächen der Vorrichtung geometrisch strukturiert ist.

19. Vorrichtung nach einem der Ansprüche 1 bis 18, **dadurch gekennzeichnet, dass** mindestens eine der sphärisch ausgebildeten Flächen der Vorrichtung aufgerauht ist.

20. Vorrichtung nach einem der Ansprüche 1 bis 19, **dadurch gekennzeichnet, dass** mindestens eines der mit einer sphärischen Fläche versehenen Teile der Vorrichtung mindestens im Bereich der sphärischen Ausbildung weicher gestaltet ist als die anderen Teile der Vorrichtung.

21. Vorrichtung nach einem der Ansprüche 1 bis 20, **dadurch gekennzeichnet, dass** das erste Teil (1) ein Knochenfixationsmittel (24) ist, so dass das untere Segment (13) zur Fixation des Knochenfixationsmittels (24) an einem Knochen dient.

## Claims

1. A device for a ball-and-socket joint type connection of two parts, comprising:
A) a first member (1) and a second member (2), which are connected with one another in the form of a ball-and-socket joint;
B) the first member (1) having a first central axis (5), a first joint segment (10) with an upper end (11) and a lower segment (13) which is unspecific with regard to the joint connection;
C) the second member (2) having a second central axis (6), a second joint segment (20) with a lower end (22) and an upper segment (23) with an upper end (21) and being unspecific with regard to the joint connection;
D) the first joint segment (10) being configured as a joint head (3) whereas the second joint segment (20) is configured as a cavity (4), which accommodates the joint head (3), the cavity (4) being concentric with the second central axis (6) and having an opening (15) open towards the lower end (22) of the second member (2) and apt for the passage of the lower segment (13) of the first member (1) therethrough; wherein
E) the cavity (4) being concentric with the second central axis (6) further includes a constriction (14) formed by a tapered surface formed in the lower end (22) of the second member;
F) the constriction (14) having a support (37) for the joint head (3), which is directed towards the interior of the cavity (4); wherein
G) at least one of the joint segments (10; 20) in the contact region of the joint segment (10; 20) is configured spherically with a joint center Z; wherein
H) the joint-like connection between the two joint segments (10; 20) is reversibly fixable by clamping means (7), said clamping means (7) including a front and a rear end (16;17) and being connectable with the second joint segment (20) and arranged coaxially with the second central axis (6);
I) the joint head (3) having a depression (8) at the upper end (11), where said front end (16) of the clamping means (7) is abuttable; and
K) the depression (8) and/or the front end (16) of the clamping means (7) includes a partially spherical surface (9), which has a center C, whereby
L) the joint center Z and the center C are identical when the device is locked;
M) the second member (2) is a connecting part (25) for connecting a longitudinal member (26) with the first member (1); and
N) the connecting part (25) including a channel (27) sized and configured to accommodate the longitudinal member (26), the channel (27) further including a channel axis (28) which is disposed transversely to the central axis (6);
**characterised in that**
O) the channel axis (28) is displaced laterally with respect to the central axis (6) such that the clamping means (7) does not protrude laterally into the channel (27); and
P) the device further comprises a covering cap (33) having a tab (34) parallel to the central axis (6), the covering cap (33) being slideable over the connecting part (25) coaxially to the second central axis (6) and being forceable towards the lower end (22) of the connecting part (25) by means of mounting means (29) arranged at the connecting part (25), whereby
Q) the tab (34) laterally surrounds a longitudinal member (26) inserted in the channel (27) when the covering cap (33) is mounted.

2. The device of claim 1, wherein the second member (2) further comprises a central borehole (30), which is disposed coaxially with the second central axis (6).

3. The device of claim 2, further comprising mounting means (29) arranged at the connecting part (25) for reversibly securing the longitudinal member (26) into the channel (27).

4. The device of claims 2 or 3, wherein the central borehole (30) has an internal thread (31) and the clamping means (7) is a clamping screw, which is sized and configured to threadedly engage the internal thread (31) formed in the connecting part (25).

5. The device of one of the claims 1 to 4, wherein the channel (27) is sidewardly opened and has a channel opening (32), which is disposed transversely to the central axis (6) and transversely to the channel axis (28).

6. The device of claim 2, wherein the clamping means (7) is axially displaceable within the central borehole (30).

7. The device of claim 2, wherein the central borehole (30) has an internal thread (31) and the clamping means (7) has an external thread (47), the internal thread (31) being sufficiently long, so that the clamping means (7) can be brought into contact with the first member (1).

8. The device of claims 5 to 7, wherein the central borehole (30), at the upper end (21) of the connecting part (25), has an internal thread (31) and that the mounting means (29) can be brought into contact with the longitudinal member (26) inserted in the channel (27).

9. The device of claims 1 to 8, wherein the connecting part (25) has an external thread (43) at its upper end (21) and that the mounting means (29) is a nut which is threadably engageable with the external threads (43) so that the mounting means (29) can be brought into contact with the longitudinal member (26) inserted in the channel (27).

10. The device of one of the claims 1 to 10, wherein the front end (16) of the clamping means (7) has a tip, which is concentric with the second central axis (6).

11. The device of one of the claims 1 to 10, wherein the front end (16) of the clamping means (7) has a flat surface, which is orthogonal to the second central axis (6).

12. The device of one of the claims 1 to 12, wherein the front end (16) of the clamping means (7) has a spherical configuration.

13. The device of claim 12, wherein the front end (16) of the clamping means (7) is spherical with the center C and has a radius R.

14. The device of one of the claims 1 to 13, wherein the depression (8) is spherical with the center C.

15. The device of claim 14, wherein the depression (8) is configured as a cone (42) having a diameter and a conical angle, the diameter of the cone (42) at the upper end of the first part (1) and the conical angle being dimensioned so that the cone (42) touches an imaginary spherical surface having the center C and a radius R.

16. The device of claim 15, wherein the depression (8) has a circular edge (39), which is concentric with the central axis (6), the circular edge (39) resting on an imaginary spherical surface having the center C and a radius R.

17. The device of one of the claims 1 to 16, wherein the joint head (3) is rigid so that, upon locking the device an expansion of the joint head (3) is not possible.

18. The device of one of the claims 1 to 17, wherein at least one of the spherically constructed surfaces of the device is structured geometrically.

19. The device of one of the claims 1 to 18, wherein at least one of the spherically constructed surfaces of the device has a roughened surface.

20. The device of one of the claims 1 to 19, wherein at least one of the members of the device, which is provided with a spherical surface, is softer, at least in the region of the spherical construction, than the other members of the device.

21. The device of one of the claims 1 to 20, wherein the first member (1) comprises bone fixation means (24), so that the lower segment (13) serves to secure the bone fixation means to a bone.

## Revendications

1. Dispositif pour relier deux éléments à la manière d'une articulation sphérique comprenant
A) un premier élément (1) et un deuxième élément (2) qui sont reliés l'un à l'autre à la manière d'une articulation sphérique, dans lequel
B) le premier élément (1) comprend un premier axe central (5), un premier segment d'articulation (10) avec une extrémité supérieure (11) et un segment inférieur (13) non spécifique à la liaison articulée ;
C) le deuxième élément (2) comprend un deuxième axe central (6), un deuxième segment d'articulation (20) avec une extrémité inférieure (22) et un segment supérieur (23) avec une extrémité supérieure (21), non spécifique à la liaison articulée ;
D) le premier segment d'articulation (10) est réalisé sous forme de tête d'articulation (3) et le deuxième segment d'articulation (20) est réalisé sous forme de cavité concentrique (4) au deuxième axe central (6), logeant la tête d'articulation (3), comprenant une ouverture (15) ouverte vers l'extrémité inférieure (22) du deuxième élément (2) pour faire passer le segment inférieur (13) du premier élément (1) ;
E) la cavité (4) concentrique par rapport au deuxième axe central (6) est rétrécie vers l'extrémité inférieure (22) du deuxième élément (2) par l'intermédiaire d'un rétrécissement (14) ; et
F) le rétrécissement (14) présente un appui (37) pour la tête d'articulation (3) tourné vers l'intérieur de la cavité (4) ;
G) au moins un des segments d'articulation (10 ; 20), dans la zone de contact des segments d'articulation (10 ; 20) est réalisé sous forme sphérique avec le centre d'articulation Z ;
H) la liaison articulée entre les deux segments d'articulation (10 ; 20) qui peut être mise en place de manière amovible à l'aide d'un moyen de serrage (7) présentant une extrémité avant et une extrémité arrière (16 ; 17), disposé coaxialement au deuxième axe central (6) et pouvant être relié au deuxième segment d'articulation (20) ;
I) la tête d'articulation (3) présente un évidement (8) au niveau de l'extrémité supérieure (11), contre lequel l'extrémité avant (16) du moyen de serrage (7) peut venir s'appuyer ; et
K) l'évidement (8) et/ou l'extrémité avant (16) du moyen de serrage (7) présente une surface (9) au moins partiellement sphérique ayant le centre C, dans lequel
L) le centre d'articulation Z et le centre C sont identiques à l'état bloqué du dispositif ;
M) le deuxième élément (2) est un élément de liaison (25) pour relier un longeron (26) au premier élément (1 ) ; et
N) l'élément de liaison (25) comprend un canal (27) disposé transversalement au deuxième axe central (6) avec un axe de canal (28) pour recevoir un longeron (26), **caractérisé en ce que**
O) l'axe de canal (28) est décalé latéralement par rapport au deuxième axe central (6) de telle manière que le moyen de serrage (7) ne dépasse pas latéralement dans le canal (27) ; et
P) le dispositif comprend un chapeau de fermeture (33), lequel peut être déplacé coaxialement au deuxième axe central (6) sur l'élément de liaison (25), peut être serré contre l'extrémité inférieure (22) de l'élément de liaison (25) à l'aide de moyens de serrage (29) disposés sur l'élément de liaison (25) et comprend une patte (34) parallèle au deuxième axe central (6), dans lequel
Q) la patte (34) entoure latéralement un longeron (26) disposé dans le canal (27) lorsque le chapeau de fermeture (33) est monté.

2. Dispositif selon la revendication 1, **caractérisé en ce que** le deuxième élément (2) comprend un alésage central (30) disposé coaxialement au deuxième axe central (6).

3. Dispositif selon la revendication 2, **caractérisé en ce que** des moyens de serrage (29) sont disposés sur l'élément de liaison (25) pour bloquer de manière amovible un longeron (26) pouvant être inséré dans le canal (27).

4. Dispositif selon la revendication 2 ou 3, **caractérisé en ce que** l'alésage central (30) présente un taraudage (31) et **en ce que** le moyen de serrage (7) est une vis de serrage, laquelle peut être vissée dans le taraudage (31) dans l'élément de liaison (25).

5. Dispositif selon l'une quelconque des revendications 1 à 4, **caractérisé en ce que** le canal (27) est ouvert sur le côté et présente sur le côté une ouverture de canal (32) disposée transversalement au deuxième axe central (6) et transversalement à l'axe de canal (28).

6. Dispositif selon la revendication 2, **caractérisé en ce que** le moyen de serrage (7) peut être déplacé axialement dans l'alésage central (30).

7. Dispositif selon la revendication 2, **caractérisé en ce que** l'alésage central (30) comprend un taraudage (31) et le moyen de serrage (7) un filetage (47), dans lequel le taraudage (31) a une longueur telle que le moyen de serrage (7) peut venir s'appuyer contre le premier élément (1).

8. Dispositif selon les revendications 5 à 7, **caractérisé en ce que** l'alésage central (30) comprend un taraudage (31) au niveau de l'extrémité supérieure (21) de l'élément de liaison (25) et **en ce que** le moyen de serrage
(29) peut venir s'appuyer contre un longeron (26) inséré dans le canal (27).

9. Dispositif selon l'une quelconque des revendications 1 à 8, **caractérisé en ce que** l'élément de liaison (25) comprend un filetage (43) au niveau de son extrémité supérieure (21) et **en ce que** le moyen de serrage (29) est un écrou, lequel peut être vissé sur le filetage (43) de sorte que le moyen de serrage (29) peut venir s'appuyer contre un longeron (26) inséré dans le canal (27).

10. Dispositif selon l'une quelconque des revendications 1 à 9, **caractérisé en ce que** le moyen de serrage (7) présente, au niveau de son extrémité avant (16), une pointe concentrique au deuxième axe central (6).

11. Dispositif selon l'une quelconque des revendications 1 à 10, **caractérisé en ce que** le moyen de serrage (7) présente, au niveau de son extrémité avant (16), une face frontale plane orthogonale au deuxième axe central (6).

12. Dispositif selon l'une quelconque des revendications 1 à 11, **caractérisé en ce que** le moyen de serrage (7), au niveau de son extrémité avant (16), est réalisé sous forme sphérique.

13. Dispositif selon la revendication 12, **caractérisé en ce que** le moyen de serrage (7), au niveau de son extrémité avant (16), est réalisé sous forme sphérique avec le centre C et le rayon R.

14. Dispositif selon l'une quelconque des revendications 1 à 13, **caractérisé en ce que** l'évidement (8) est réalisé sous forme sphérique avec le centre C.

15. Dispositif selon la revendication 14, **caractérisé en ce que** l'évidement (8) est réalisé sous forme de cône (42), dans lequel le diamètre du cône (42) au niveau de l'extrémité supérieure du premier élément (1) et l'angle de cône sont dimensionnés de telle manière que le cône (42) touche une surface sphérique imaginaire avec le centre C et le rayon R.

16. Dispositif selon la revendication 15, **caractérisé en ce que** l'évidement (8) est pourvu d'une arête circulaire (39) concentrique au deuxième axe central (6), dans lequel l'arête (39) est située sur une surface sphérique imaginaire avec le centre C et le rayon R.

17. Dispositif selon l'une quelconque des revendications 1 à 16, **caractérisé en ce que** la tête d'articulation (3) est rigide, de sorte que, lors du blocage du dispositif, aucun élargissement de la tête d'articulation (3) n'est possible.

18. Dispositif selon l'une quelconque des revendications 1 à 17, **caractérisé en ce qu'**au moins une des surfaces sphériques du dispositif présente une texture géométrique.

19. Dispositif selon l'une quelconque des revendications 1 à 18, **caractérisé en ce qu'**au moins une des surfaces sphériques du dispositif est rendue rugueuse.

20. Dispositif selon l'une quelconque des revendications 1 à 19, **caractérisé en ce qu'**au moins un des éléments du dispositif pourvus d'une surface sphérique est plus souple, au moins dans la zone de la formation sphérique, que les autres éléments du dispositif.

21. Dispositif selon l'une quelconque des revendications 1 à 20, **caractérisé en ce que** le premier élément (1) est un moyen permettant de fixer un os (24), de sorte que le segment inférieur (13) sert à la fixation sur un os du moyen permettant de fixer un os (24).
